# EUROPEAN PATENT SPECIFICATION

(11) **EP 4 322 866 B1**
(45) Date of publication and mention of the grant of the patent: **23.07.2025**
(21) Application number: 21720220.9
(22) Date of filing: 16.04.2021
(51) Int. Cl.: A61B 17/12

(54) **EMBOLISATION SYSTEM FOR PROMOTING CLOT FORMATION**
EMBOLISIERUNGSSYSTEM ZUR FÖRDERUNG DER GERINNSELBILDUNG
SYSTÈME D'EMBOLISATION PERMETTANT DE FAVORISER LA FORMATION DE CAILLOT

(43) Date of publication of application: 21.02.2024
(73) Proprietor: Clearstream Technologies Limited, Enniscorthy, County Wexford (IE)
(72) Inventor: RONAN, Allan, Enniscorthy, County Wexford (IE); WALL, Sean, Enniscorthy, County Wexford (IE); SHERIDAN, Stephen, Enniscorthy, County Wexford (IE); GILES, Ciaran, Enniscorthy, County Wexford (IE)
(74) Representative: Hoffmann Eitle
(86) International application number: PCT/EP2021/059918
(87) International publication number: WO 2022/218541

(56) References cited:
- EP-A1- 3 756 591
- WO-A1-2021/013352
- US-A1- 2014 276 388
- US-B2- 10 675 039

## Description

### Technical field

The present disclosure relates to an apparatus comprising an embolisation device for promoting clot formation in a bodily lumen, the embolisation device having a collapsed delivery configuration and an expanded deployed configuration for anchoring the embolisation device in the bodily lumen. More particularly, the present disclosure relates to an apparatus comprising a recapture mechanism which is configured to move a set of flexible bristles of the embolisation device to a lower radial profile than an expanded deployed configuration. The present disclosure also relates to a method of manufacturing the apparatus.

### Background

Embolisation devices may be deployed in the vasculature at a particular location by a medical practitioner so as to promote blood clot formation and ultimately occlude the blood vessel. Typically, an embolisation device is pushed through a guide (delivery) catheter in a distal direction using a delivery wire until a point of deployment within a bodily lumen is reached. Once the device reaches the required point of deployment, the device is deployed from the guide catheter.

In some cases, the embolisation device may need to be recaptured after deployment from the delivery catheter, for example if the final position of the embolisation device within the bodily lumen is incorrect and needs to be re-adjusted.

In cases where the embolisation device must be recaptured, the delivery catheter must be moved back over the embolisation device.

The embolisation device may comprise a group of flexible bristles that extend radially outwardly and longitudinally in a direction opposite to the delivery direction of the embolisation device (i.e. a proximal direction). As the delivery catheter is moved over the embolisation device in the distal direction, the movement of the delivery catheter along the bristles can cause some or all of the flexible bristles in this group move longitudinally in the distal direction. Recapture of the embolisation device can therefore cause the proximally-orientated bristles to extend instead in the distal direction, leading to misalignment of the bristles. Such a misalignment can reduce the effectiveness of the embolisation device, such as increasing the time to occlusion of a blood vessel.

In view of the above, there is a need for an improved embolisation device which is able to be recaptured without causing damage to the proximally-oriented bristles, or without reorienting them.

WO 2021/013352 A1 discloses an embolisation device for promoting clot formation in a bodily lumen and having a collapsed delivery configuration for delivery of the embolisation device into, and retrieval of the embolisation device from, the bodily lumen and an expanded deployed configuration for anchoring the embolisation device in the bodily lumen. The embolisation device comprises a tubular cage having a collapsed delivery configuration and an expanded deployed configuration. The embolisation device further comprises an embolisation member disposed in the tubular cage, the embolisation member comprising a stem and a plurality of flexible bristles extending outwardly from the stem, the plurality of flexible bristles having a collapsed delivery configuration and an expanded deployed configuration. The embolisation device is configured such that as the tubular cage is transitioned from its expanded deployed configuration to its collapsed delivery configuration the plurality of flexible bristles is urged by the tubular cage from the expanded deployed configuration of the flexible bristles to the collapsed delivery configuration of the flexible bristles.

US 2014/276388 A1 discloses a device for delivery into a body lumen having a longitudinally extending stem and a plurality of bristles extending generally radially outwardly from the stem. The device includes at least two different groups or types of bristles.

### Summary

According to a first aspect, there is provided an apparatus comprising an embolisation device for promoting clot formation in a bodily lumen, the embolisation device comprising a core and a plurality of flexible bristles extending outwardly from the core, the bristles having a collapsed delivery configuration and an expanded deployed configuration in which the bristles extend at least radially outwardly from the core to contact the lumen and to anchor the device in the lumen, and a delivery catheter, wherein: the embolisation device is configured to be delivered in a distal direction from the delivery catheter to the lumen; in the expanded deployed configuration, a set of the flexible bristles extend radially outwardly from the core and longitudinally in a proximal direction opposite to the distal direction; and the apparatus further comprises a recapture mechanism, actuatable independently of the movement of the delivery catheter over the bristles, for moving the set of flexible bristles to a lower radial profile than in the expanded deployed configuration before the delivery catheter is moved over the set of flexible bristles.

According to a second aspect, there is provided an embolisation system comprising the embolisation device of the first aspect; and a delivery element for delivering the embolisation device to a bodily lumen through the delivery catheter; wherein the recapture mechanism is actuatable to decrease the radial profile the set of flexible bristles to a radial profile smaller than an inner radius of the distal tip of the delivery catheter.

According to a third aspect, there is provided a method of manufacturing an apparatus comprising an embolisation device for promoting clot formation in a bodily lumen, the embolisation device comprising a core and a plurality of flexible bristles extending outwardly from the core, the bristles having a collapsed delivery configuration and an expanded deployed configuration in which the bristles extend at least radially outwardly from the core to contact the lumen and to anchor the device in the lumen, the method comprising: providing a delivery catheter; providing a core; providing a set of flexible bristles configured to extend radially outwardly from the core and longitudinally in a proximal direction opposite to a direction in which the embolisation device is configured to be delivered from a delivery catheter; and providing a recapture mechanism, actuatable independently of the movement of the delivery catheter over the bristles, for moving the set of bristles to a lower radial profile than an expanded deployed configuration of the set of bristles before the delivery catheter is moved over the set of flexible bristles.

### Brief description of the drawings

To enable better understanding of the present disclosure, and to show how the same may be carried into effect, reference will now be made, by way of example only, to the accompanying drawings, in which:
Fig. 1A shows an exemplary embolisation device in an unconstrained configuration;
Fig. 1B shows the exemplary embolisation device of Fig. 1A in a collapsed delivery configuration;
Fig. 1C shows the exemplary embolisation device of Fig. 1A in an expanded deployed configuration;
Fig. 1D shows an exemplary joint between bristle segments of an embolisation device;
Fig. 1E shows an exemplary joint between bristle segments of an embolisation device;
Fig. 1F shows an exemplary joint between bristle segments of an embolisation device;
Fig. 2A shows an apparatus comprising an embolisation device according to one or more embodiments shown and described herein;
Fig. 2B shows the apparatus of Fig. 2A in another configuration;
Fig. 2C shows the apparatus of Fig. 2A in another configuration;
Fig. 3A shows an apparatus comprising an embolisation device according to one or more embodiments shown and described herein;
Fig. 3B shows the embolisation device of Fig. 3A in another configuration;
Fig. 4A shows another apparatus comprising an embolisation device according to one or more embodiments shown and described herein;
Fig. 4B shows the embolisation device of Fig. 4A in another configuration;
Fig. 5A shows another apparatus comprising an embolisation device according to one or more embodiments shown and described herein;
Fig. 5B shows the embolisation device of Fig. 5A in another configuration;
Fig. 6 shows another apparatus comprising an embolisation device according to one or more embodiments shown and described herein;
Fig. 7 shows the apparatus of Fig. 6 in another configuration;
Fig. 8 shows another apparatus comprising an embolisation device according to one or more embodiments shown and described herein;
Fig. 9 shows another apparatus comprising an embolisation device according to one or more embodiments shown and described herein;
Fig. 10 shows another apparatus comprising an embolisation device according to one or more embodiments shown and described herein;
Fig. 11A shows a linking element attached to a bristle by a heat-shrinkable element, according to one or more embodiments; and
Fig. 11B shows an elongate element slidably connected to a bristle by a loop, according to one or more embodiments.

### Detailed description

Throughout this disclosure the term 'embolisation device' may refer to a device which may be permanently or semi-permanently implanted in a bodily lumen. Accordingly, the 'embolisation device' may be configured to be disposed within the bodily lumen for a period of time, such as a number of days, or disposed in the lumen indefinitely. To this end, the 'embolisation device' may be configured to be selectively detached from a delivery element so that it may be implanted in the bodily lumen in isolation.

Throughout this disclosure the term 'bodily lumen' may refer to the inside space within a tubular structure of the human or animal body. The 'bodily lumen' may be, for example, an artery or vein.

Throughout this disclosure the term 'contracted delivery configuration' of an element may refer to a configuration of the element which has a smaller radial extent than an expanded deployed configuration of the element.

Throughout this disclosure the term 'to anchor' may refer to partly or fully securing an element in a position.

Throughout this disclosure, the term 'core' may refer to an element which acts as a backbone for the device. The term 'stem' may refer to an elongate element which extends longitudinally along the length of the embolisation device to act as a backbone for the device, and has a significantly smaller radial extent than the further elements of the embolisation device (for example, the plurality of flexible bristles). The stem may extend along substantially the whole longitudinal extent of the plurality of flexible bristles (e.g. when the embolisation device is in an unrestrained configuration, contracted delivery configuration and/or expanded deployed configuration). The stem may extend along substantially the whole length of the embolisation device.

In any of the examples described herein, the term 'bristle' may refer to an elongate strand of material formed substantially a single piece. The 'bristle' may be a resilient bristle. The resilient bristle may be biased towards a particular curvature.

Throughout this disclosure, the term 'radially outwardly' does not exclude the element additionally extending in the longitudinal direction of the device. For example, the plurality of flexible bristles may extend radially outwardly and longitudinally from the stem.

Through this disclosure, the term 'radial profile' may refer to a radial extent in a particular direction radially outwardly from the stem of the embolisation device. For example, the embolisation device has a lower radial extent in the contracted delivery configuration than in the expanded deployed configuration.

The plurality of flexible bristles may have a contracted configuration in the contracted delivery configuration. The plurality of flexible bristles may have an expanded configuration in the expanded deployed configuration. The plurality of bristles may extend radially outwardly from the stem in a plurality of circumferential directions about the stem.

In the expanded configuration, the plurality of flexible bristles may be configured to anchor the device in the bodily lumen. The plurality of flexible bristles may be configured to provide substantially all of the anchoring force for the embolisation device in the bodily lumen.

In the expanded configuration, the plurality of flexible bristles may be configured to contact the bodily lumen.

In the contracted delivery configuration, the plurality of flexible bristles extending outwardly from the stem are contracted such that radial extent of the device is less than the radial extent of the expanded deployed configuration of the element. In the contracted delivery configuration, the device fits inside the delivery catheter.

The bristles may be made of a flexible material such as stainless steel, Elgiloy or Nitinol. Other suitable materials may also be used, such as any suitable polymer or any other shape memory metal or metal alloy. The flow restrictor may be a membrane or a thin film membrane made of a self-expanding material such as a polymer, stainless steel or Nitinol. The core may be made of stainless steel or other suitable material and may comprise a twisted wire from which the bristles extend and on which the flow restrictor is mounted. The stem may alternatively comprise a hollow tube wherein the walls of the hollow tube hold the radially extending bristles in place. For example, the tube may be formed from a heat shrinkable material. Alternatively or additionally, the bristles may be held by the stem using other means such as adhesives.

The diameter of an individual flexible bristle may range from about 0.036mm (0.0014 inches) to about 0.053mm (0.0021 inches). For example, the diameter of an individual flexible bristle may be about 0.381mm (0.015 inches), about 0.445mm (0.0175 inches) or about 0.508mm (0.02 inches). The overall radial diameter of the expanded flexible bristles may range from about 5mm to about 30mm.

A flow restrictor may be a membrane made from thin film Nitinol, thin film PTFE, a thin film elastomer such as polyurethane or any other type of suitable biocompatible material. The membrane 36 may have a thickness of about 4µm to about 35µm and a radial diameter of about 5mm to about 20mm. For example, the diameter of the membrane 36 may be about 6.5mm, about 9mm or about 16mm. Furthermore, the membrane 36 may be non-permeable or semi-permeable.

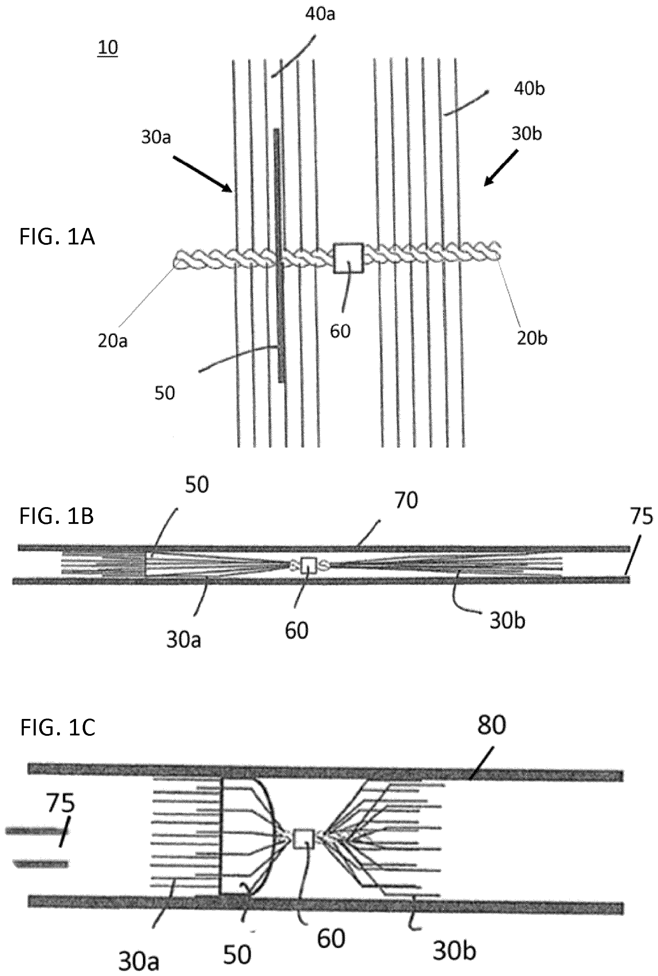

Fig. 1A shows an exemplary embolization device 10 in an unconstrained configuration. The embolization device 10 comprises a plurality of flexible bristles 40a, 40b having deployed and collapsed configurations. The device may comprise a series of segments (for example, two) wherein the bristles 40a of at least one segment 30a are configured to point distally when deployed in a bodily lumen, and the bristles 40b of at least one segment 30b are configured to point proximally when deployed in a bodily lumen. In some cases, there is only a proximal segment 30a and distal segment 30b. The bristles of each segment 30a, 30b extend radially outwardly from a core, and more specifically stems 20a, 20b.

A proximally pointing segment is defined as a segment in which the bristles point proximally. The proximally pointing segment 30a may comprise a flow restrictor 50. The flow restrictor may be a cone-shaped membrane open at the proximal end or may be another shape such as disc shaped. The flow restrictor 50 comprises a hole (not shown) through which the stem 20a passes such that the flow restrictor 50 is mounted on the stem 20a. A distally pointing segment is defined as a segment in which the bristles point distally. The flow restrictor 50 may alternatively be present on the distally pointing segment and may be a cone-shaped membrane open at the distal end or may be another shape such as a disc shape.

In some examples, at least one segment, such as in the illustrated case the proximal segment 30a, incorporates a flow restrictor 50. The flow restrictor 50 is configured to restrict blood flow in the vessel when the device 10 is deployed.

In some examples a series of radiopaque markers divides the proximally pointing segment 30a and the distally pointing segment 30b, such as the radiopaque marker 60 shows in FIG. 1A. The radiopaque marker 60 may alternatively be positioned proximally to the most proximal segment or distally to the most distal segment, or in between segments. The device may comprise one or more markers 60. There may be a proximal marker (not depicted) positioned proximal to the proximal bristle segment 30a, a distal marker (not depicted) distal to the distal segment 30b, and/or an intermediate marker 60 positioned between the proximal bristle segment and the distal segment. In other examples, the device does not comprise any markers.

In some examples the bristles 40a, 40b are formed on respective stems 20a, 20b. In the illustrated example the stems 20a, 20b comprise a twisted metal wire which securely holds the bristles in the twists of the wire. The stems 20a, 20b may alternatively comprise a hollow tube wherein the walls of the hollow tube hold the radially extending bristles in place. For example, the tube may be formed from a heat shrinkable material or a meltable material. Alternatively or additionally, the bristles may be held by the stem using other means such as adhesives.

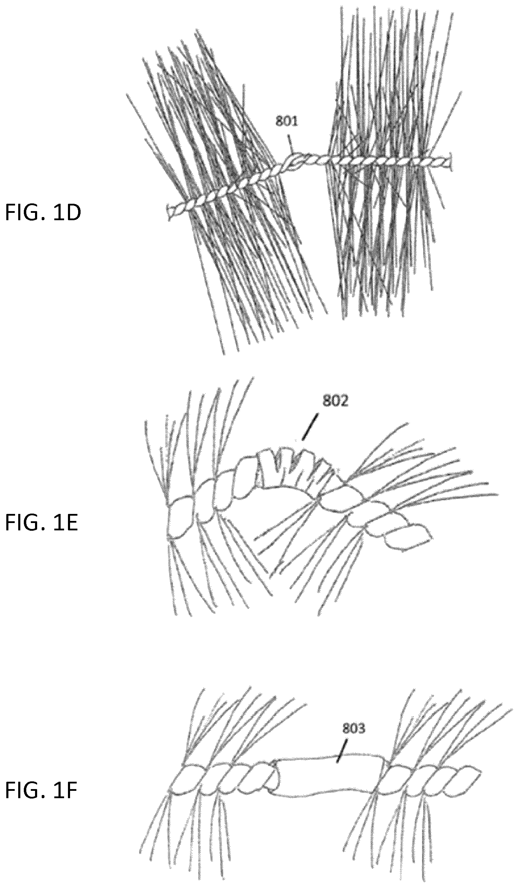

The stems 20a, 20b may be separately formed and joined by an articulating, flexible or non-articulating joint. For example, in the illustrated example the stems 20a, 20b are connected via the radiopaque marker 60 which is securely fastened to the stems 20a, 20b and which is made of an inflexible material. Alternatively, the marker 60 may merely be a radiopaque band situated at a location on one of the stems or the joint, and the stems 20a, 20b may be joined by an articulating joint 801 such as that shown in Fig. 1D, a flexible joint 802 such as the flexible tube 802 of Fig. 1E which comprises slots to accommodate bending of the tube, or an inflexible joint 803 such as that shown in Fig. 1F which may be a crimping connector. In other examples, the bristle segments may be formed on a single stem and the stem may be more or less flexible at the section joining the bristle segments.

In one case the embolization device comprises only a single proximal segment 30a and a single distal segment 30b. The proximal segment 30a and the distal segment 30b in one case are mounted on a single common stem. The stem 20a of the proximal segment 30a and the stem 20a of the distal segment 30b may form parts of the same continuous stem.

In the case where the device comprises more than two segments, the connection between the two most proximal segments may be stiffer than the distal connections. The distal connections may generally comprise a hinge.

In one example, a flow restrictor 50 comprising a flexible membrane is present in at least one of the segments. The membrane may comprise a disc of thin film material. The flexibility of the membrane means its orientation is controlled by the orientation of the adjacent bristles - in the illustrated example, if the adjacent bristles 40a are forced to point distally the membrane will adjust its configuration accordingly. Thus, if the membrane is deployed from a collapsed condition, such as from within a catheter, the bristles will cause it to open up to an expanded configuration. The membrane may also be placed proximal or distal to the segment.

Optionally, the membrane may comprise lines along which the membrane preferentially collapses when it is in the collapsed configuration, so that folding of the membrane in the collapsed configuration is controlled in a predictable manner.

In one case, the embolization device 10 comprises at least two segments. In one configuration the flow restrictor 50 is in the most proximal segment. This is shown in an unconstrained state schematically in Fig. 1A. In the configuration shown the flow restrictor 50 is located within the proximal segment 30a with bristles 40a both proximal and distal to the flow restrictor 50. In some cases, there may be a distal flow restrictor (not shown), and there may be any number of flow restrictors provided on the embolization device.

The flow restrictor 50 may have an outer dimension which is less than an outer dimension of the plurality of anchoring bristles. The flow restrictor may be connected to the stem. In some examples the flow restrictor may have a central hole that is an interference fit on the stem. The central hole in the flow restrictor is preferably smaller than the stem on which it is mounted. The central hole in the flow restrictor may have a diameter which is smaller than the diameter of the stem prior to assembly of the device.

The device 10 has a collapsed configuration to facilitate delivery through a catheter. By placing the flow restrictor 50 within the bristles 40a of the segment 20a, i.e. with bristles proximal and distal to it, it is protected from damage while the implant is being collapsed or pushed through a catheter. Furthermore, any friction between the catheter and the flow restrictor 50 is reduced.

In one configuration, the implant is collapsed such that the bristles 40a of the most proximal segment 30a point proximally, while the bristles 40b of the distal segment 30b or segments point distally. Since the flow restrictor orientation is controlled by the orientation of the bristles, if the flow restrictor 50 is within the proximal segment, it will also point proximally. This is shown schematically in Fig. 1B, with the device 10 shown in a collapsed delivery configuration within a delivery catheter 70.

A distal end 75 of the catheter 70 is positioned at a target location within a bodily lumen, and the embolization device 10 is delivered from the catheter 70 into the bodily lumen from the distal end. For example, a delivery element (not shown) may be used to push the embolization device 10 through the delivery catheter 70. The delivery element may be moved distally relative to the delivery catheter 70 to deliver the embolization device 10 from the distal end 75.

Fig. 1C shows the embolization device 10 in an expanded deployed configuration within a bodily lumen 80, after being delivered from the distal end 75 of the delivery catheter 70. The plurality of flexible bristles anchor the embolization device 10 to the bodily lumen 80. A set of bristles 40a are configured to extend radially outwardly from the core and longitudinally in a proximal direction opposite to the distal direction
It can be seen from Fig. 1C that once the proximal segment 30a is in the expanded deployed configuration, it is difficult to recapture the embolisation device 10. In particular, if the delivery catheter 75 is advanced over the proximal bristle segment 30a, the bristles of the proximal segment 30a are forced by the delivery catheter 75 into a distal orientation. This can cause damage to the bristles and unwanted reorientation of the bristles. Thus, recapture and redeployment of the embolisation device 10 affects the correct functioning of the device in its redeployed position.

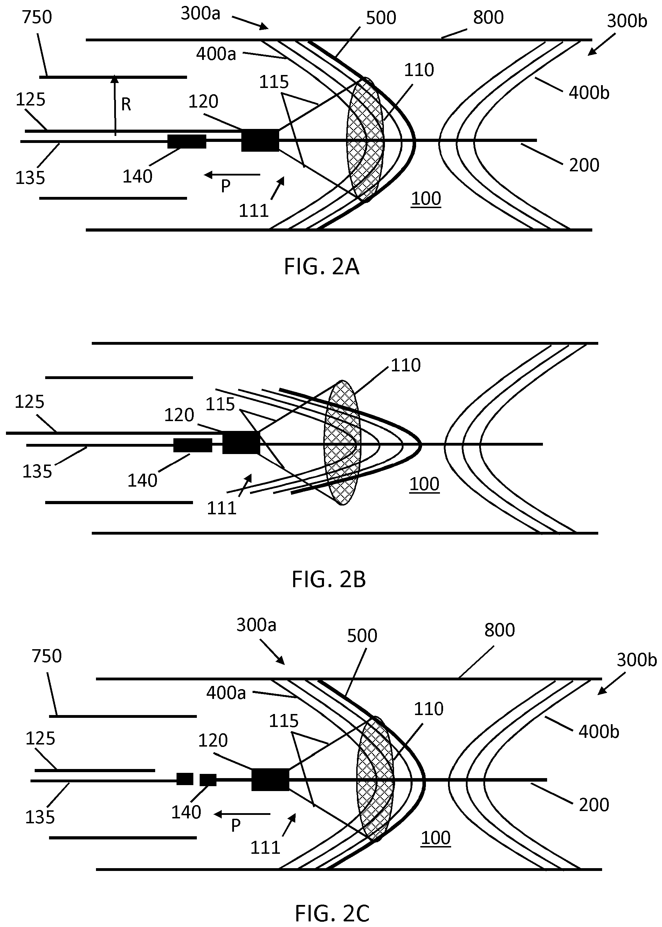

Fig. 2A shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 2A, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

In the embodiment shown in Fig. 2A, the apparatus comprises a mesh 110 mounted on the core 200 and connected to the set of bristles 400a radially outwardly from the core 200. The mesh 110 may be fixedly mounted to the core 200 (e.g. by welding or adhesive or a frictional fit) or it may be slidably mounted to the core 200. The set of bristles 400a may be fixedly connected to the mesh 110 (e.g. by welding or adhesive to the mesh 110 at points on the bristles 400a radially outwardly from the core 200), or may be slidably connected to the mesh 110 (for example the bristles 400a may pass through holes in the mesh 110 from one longitudinal side of the mesh 110 to the other longitudinal side of the mesh 110. The mesh 110 is configured to be retracted by the recapture mechanism 111 in the proximal direction P. In doing so, the bristles 400a are pulled in the proximal direction P and move to a lower radial profile as shown in Fig. 2B.

In the embodiment show in Figs. 2A to 2C, the recapture mechanism 111 comprises a sliding element 120 slidably mounted on the core 200 proximal to the set of bristles 400a and coupled to the mesh 110 via linking elements 115 (for example metallic or polymer wires whose respective ends are attached to the sliding element 120 and the mesh 110). The sliding element 120 may be, for example, a collar, tube, ring or similar slidably mounted on the core 200. The sliding element 120 is connected to an elongate element 125 (for example a wire, line, ribbon, or similar element configured to extend inside the delivery catheter along some or all the length of the delivery catheter) which extends through the delivery catheter 750 and is retractable by a user of the apparatus by a control mechanism. In all embodiments disclosed herein, the control mechanism may be any mechanism which is controllable by the user to retract (and optionally advance) the elongate element(s) through the delivery catheter. For example, the control mechanism may comprise a spool connected to the proximal end of the elongate element(s), rotatable by the user, either manually or by a motor, to wind the elongate element(s). As the elongate element(s) are wound, the elongate element(s) are retracted. The elongate element 125 may instead be retracted manually by the user by pulling a proximal end of the elongate element 125 in a proximal direction. The elongate element may be, for example, made of nitinol.

In some embodiments, the embolisation device 100 comprises a detach element 140 which is configured to removably connect to a delivery element 135. In all embodiments disclosed herein, the detach element 140 may be any element which removably connects the embolisation device 100 to the delivery element 135. The detach element 140 may for example be a male or female screw thread on the embolisation device 100 configured to removably attach to a counterpart female or male screw thread on the delivery element 135. The detach element 140 may be an electrolytic element (formed of, e.g. formed of platinum, stainless steel, nitinol and/or cobalt chromium) configured to disintegrate upon application of an electric current delivered to the electrolytic element via the delivery element 135. The detach element 140 may be a connector between the embolisation device 100 and the delivery element 135 which is configured to break after a predetermined number of rotations. In all embodiments disclosed herein, the delivery element 135 may be any elongate element such as a wire, line or ribbon configured to extend through the delivery catheter 750. The delivery element 135 is operable to push the embolisation device 100 through the delivery catheter 750. The embolisation device 100 is deployed to the expanded deployed configuration by moving the delivery element 135 distally relative to the delivery catheter (for example by holding the delivery catheter 750 and pushing the delivery element 135 or by holding the delivery element 135 and pulling the delivery catheter). If the embolisation device 100 has been deployed in the bodily lumen 800 (for example as shown in Fig. 2A) and must be recaptured, a user of the apparatus may actuate the recapture mechanism 111 by retracting the elongate element 125 in the proximal direction P (and holding the delivery element 135 in place), which retracts the sliding element 120 and therefore the mesh 110 via the linking elements 115. Doing so causes the bristles 400a to be moved to a lower radial profile as shown in Fig. 2B. The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius R of the delivery catheter 750 so that they do not abut the delivery catheter 750 when being recaptured.

Once the embolisation device 100 is recaptured, the delivery catheter 750 may be repositioned and the embolisation device 100 may be redeployed (for example as shown in Fig. 2A, at a different position in the lumen 800). The recapture mechanism 111 may be reversed (i.e. advanced) to redeploy the bristles 400a or the bristles 400a may have a stiffness which reverse (i.e. advance) the recapture mechanism 111 such that the bristles are redeployed if the recapture mechanism 111 is not being actuated. The elongate element 125 may be detached from the sliding element 120 and the delivery element 135 may be detached from the detach element 140. The elongate element 125 and the delivery element 135 may be configured to detach by any suitable detachment mechanism. For example, the elongate element 125 and/or the delivery element 135 may comprise a male or female screw thread configured to connect to a corresponding female or male screw thread of the sliding element 120/detach element 140. Accordingly, the elongate element 125 and/or delivery element 135 may be rotated in order to detach from the embolisation device. Alternatively, the elongate element 125 and/or the delivery element 135 may be attached to the sliding element 120 and detach element 140, respectively, and configured to break after a predetermined number of twists/rotations of the element so that they detach from the embolisation device 100. In another alternative, the elongate element 125 and/or delivery element 135 may be attached to the sliding element 120/detach element 140 by an electrolytic element which is operable to disintegrate by electrolysis in the bodily lumen by applying an electric current to the electrolytic element. The electrolytic element may be formed of platinum, stainless steel, nitinol and/or cobalt chromium.

Fig. 2C shows the apparatus when the elongate element 125 and delivery element 135 have been detached from the embolisation device 100. The delivery catheter 750, elongate element 125 and delivery element 135 may then be removed and the embolisation device 100 is deployed.

In embodiments where the mesh 110 is slidably connected to both the stem 200 and the bristles 400a, the elongate element 125 may remain connected to the mesh 110 when being retracted. In particular, the elongate element 125 may retract the sliding element 120 and the mesh 110 off the embolisation device 100 such that the mesh 110 is removed from the embolisation device 100 when it is deployed. Accordingly, the mesh 110 may remain part of the embolisation device 100 when implanted in the lumen 800 or it may be removed completely.

The mesh 110 may also be connected to a flow restricting membrane 500 attached to the core 200, having a collapsed delivery configuration and an expanded deployed configuration. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In the embodiment shown in Figs. 2A to 2C, the flow restricting membrane 500 is attached to the mesh (e.g. by welding or adhesive) and is therefore also retracted to a lower radial profile when the mesh 110 is retracted. In other embodiments the flow restricting membrane is not present or is not connected to the mesh 110, and may be configured to extend longitudinally in a distal direction.

For any embodiments disclosed herein, the mesh 110 may be made of a woven metal or polymer. The mesh 110 may be made of a shape memory material such as nitinol. The mesh 110 may be made of a heat-shrinkable material or a meltable polymer, and may be heat-shrunk or melted onto the bristles such that the bristles 400a are securely attached to the mesh 110. Alternatively, the bristles may be attached to the mesh 110 by welding or adhesive. Preferably, the radial profile of the mesh 110 in an unconstrained configuration is less than the inner radius of the delivery catheter 750.

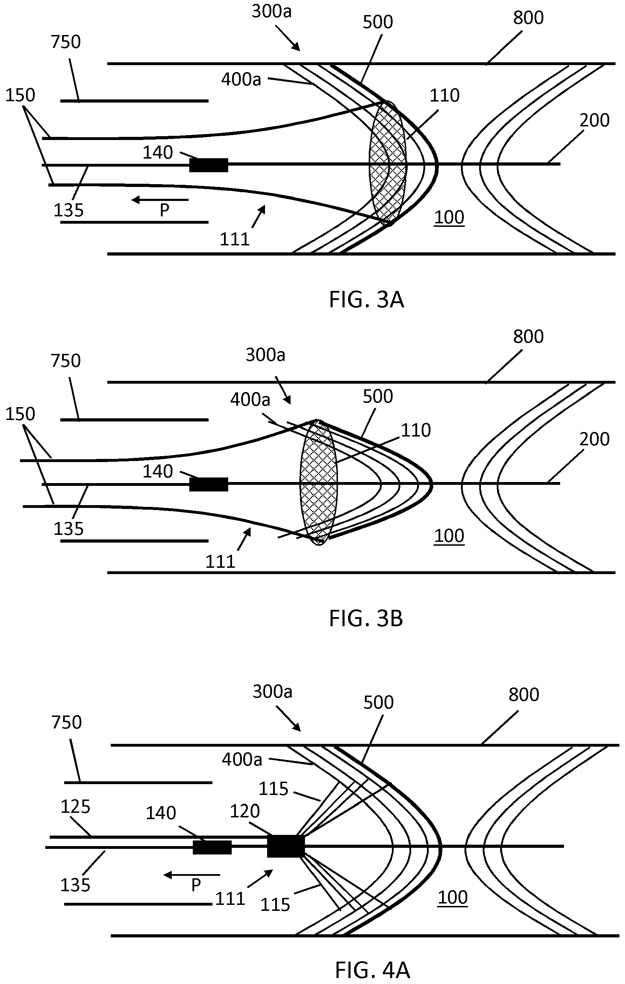

Fig. 3A shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 3A, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

Once again, the apparatus comprises a mesh 110 mounted on the core 200 and connected to the set of bristles 400 radially outwardly from the core 200. The mesh 110 may be fixedly mounted to the core 200 (e.g. by welding or adhesive or a frictional fit) or it may be slidably mounted to the core 200. The set of bristles 400a may be fixedly connected to the mesh 110 (e.g. by welding or adhesive to the mesh 110 at points on the bristles 400a radially outwardly from the core 200), or may be slidably connected to the mesh 110 (for example the bristles 400a may pass through holes in the mesh 110 from one longitudinal side of the mesh 110 to the other longitudinal side of the mesh 110. The mesh 110 is configured to be retracted by the recapture mechanism 111 in the proximal direction P and moved to a lower radial profile.

In the embodiment shown in Fig. 3A, the recapture mechanism 111 comprises one or more retractable elongate elements 150 attached to the mesh 110 and therefore coupled to the bristles 400a via the mesh 110. The elongate elements 150 traverse the length of the delivery catheter 750 and are operable to be retracted by a user via a control mechanism.

Again, the embolisation device 100 may comprise a detach element 140 configured to removably connect to a delivery element 135. The delivery element 135 is operable to deliver the embolisation device through the delivery catheter 750 as described with reference to Figs. 2A to 2C. If the embolisation device 100 has been deployed in the bodily lumen 800 but must be recaptured, a user of the apparatus may actuate the recapture mechanism 111 by retracting the elongate elements 150 in the proximal direction P (and holding the delivery element 135 in place) which retracts the mesh 110. Doing so causes the bristles 400a to be moved to a lower radial profile (shown in Fig. 3B). The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius of the delivery catheter 750 so that they do not abut the delivery catheter 750 when being recaptured. Again, once recaptured, the embolisation device 100 may be redeployed at a new position in the lumen 800. The recapture mechanism 111 may be reversed (i.e. advanced) to redeploy the bristles 400a or the bristles 400a may have a stiffness which reverse i.e. advance the recapture mechanism 111 such that the bristles are redeployed if the recapture mechanism 111 is not being actuated. The elongate elements 150 may be detached from the mesh 110 and the delivery element 135 may be detached from the detach element 140. The elongate elements 150/delivery element 135 may be removably connected to the mesh 110/detach element 140 by any suitable mechanism. For example, as described above, they may be connected via a screw mechanism, or configured to break off from the mesh 110/detach element 140 after a predetermined number of twists/rotations, or they may be attached to the mesh 110/detach element 140 via an electrolytic element configured to disintegrate upon application of an electric current through the elongate elements 150/delivery element 135.

As in the case of the embodiments described with reference to Figs. 2A to 2C, in embodiments where the mesh 110 is slidably connected to both the stem 200 and the bristles 400a, the elongate elements 150 may remain connected to the mesh 110 when being retracted. In particular, the elongate elements 150 may retract the mesh 110 off the embolisation device 100 such that the mesh 110 is removed from the embolisation device 100 when it is deployed. Accordingly, the mesh 110 may remain part of the embolisation device 100 when implanted in the lumen 800 or it may be removed completely.

The mesh 110 may also be connected to a flow restricting membrane 500 attached to the core 200, having a collapsed delivery configuration and an expanded deployed configuration. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In the embodiment shown in Fig. 3A, the flow restricting membrane 500 is attached to the mesh (e.g. by welding or adhesive) and is therefore also retracted to a lower radial profile when the mesh 110 is retracted. In other embodiments the flow restricting membrane is not present or is not connected to the mesh 110, and may be configured to extend longitudinally in a distal direction.

The mesh 110 may be made of a metal or polymer. The mesh 110 may be made of a shape memory material such as nitinol. The mesh 110 may be made of a heat-shrinkable material or a meltable polymer, and may be heat-shrunk or melted onto the bristles such that the bristles 400a are securely attached to the mesh 110. Alternatively, the bristles may be attached to the mesh 110 by welding or adhesive.

Fig. 4A shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 4A, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

In the embodiment shown in Fig. 4A, the apparatus does not comprise a mesh. Rather, the recapture mechanism 111 comprises a sliding element 120 slidably mounted on the core 200 proximal to the set of bristles 400a and coupled to the bristles 400a via linking elements 115 (for example wires whose respective ends are attached to the sliding element 120 and the bristles 400a). The linking elements 115 may each be fixedly attached to one or more bristles at a point radially outwardly from the core 200, or they may be slidably connected to the bristles (for example the linking elements may comprise loops slidably receiving one or more bristles 400a through the loops). The sliding element 120 may be, for example, a collar slidably mounted on the core 200. The sliding element 120 is connected to an elongate element 125 which extends through the delivery catheter 750 and is retractable by a user of the apparatus via a control mechanism.

Again, the embolisation device 100 may comprise a detach element 140 configured to removably connect to a delivery element 135. The delivery element 135 is operable to deliver the embolisation device through the delivery catheter 750 as described with reference to Figs. 2A to 2C.

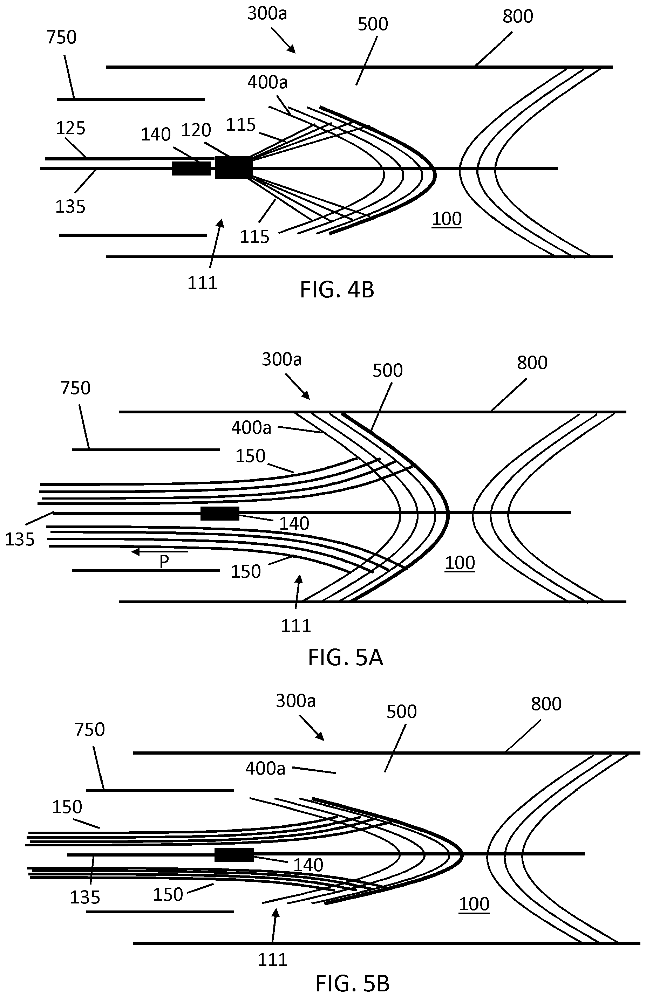

If the embolisation device 100 has been deployed in the bodily lumen 800 but must be recaptured, a user of the apparatus may actuate the recapture mechanism 111 by retracting the elongate element 125 in the proximal direction P (and holding the delivery element 135 in place) which retracts the sliding element 120. Doing so pulls the linking elements 115 in a proximal direction, which causes the bristles 400a to be moved to a lower radial profile (shown in Fig. 4B). The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius of the delivery catheter 750 so that they do not abut the delivery catheter 750 when being recaptured. Again, once recaptured, the embolisation device 100 may be redeployed at a new position in the lumen 800. The recapture mechanism 111 may be reversed (i.e. advanced) to redeploy the bristles 400a or the bristles 400a may have a stiffness which reverse (i.e. advance) the recapture mechanism 111 if the recapture mechanism 111 such that the bristles are redeployed is not being actuated. The elongate element 125 may be detached from the sliding element 120 and the delivery element 135 may be detached from the detach element 140. The elongate element 125/delivery element 135 may be removably connected to the sliding element 120/detach element 140 by any suitable mechanism. For example, as described above, they may be connected via a screw mechanism, or configured to break off from the sliding element 120/detach element 140 after a predetermined number of twists/rotations, or they may be attached to the sliding element 120/detach element 140 via an electrolytic element configured to disintegrate upon application of an electric current through the elongate element 125/delivery element 135.

In embodiments where the linking elements 115 are slidably connected to the bristles 400a, the elongate element 125 may remain connected to the sliding element 120 when the elongate element 125 is retracted. In particular, the elongate element 125 may slide the linking elements 115 off the bristles 400a and the sliding element 120 off the core 200 (and over detach element 140) such that the sliding element 120 and linking elements 115 are removed from the embolisation device 100 when it is deployed. Accordingly, the sliding element 120 and linking elements 115 may remain part of the embolisation device 100 when implanted or may be removed completely.

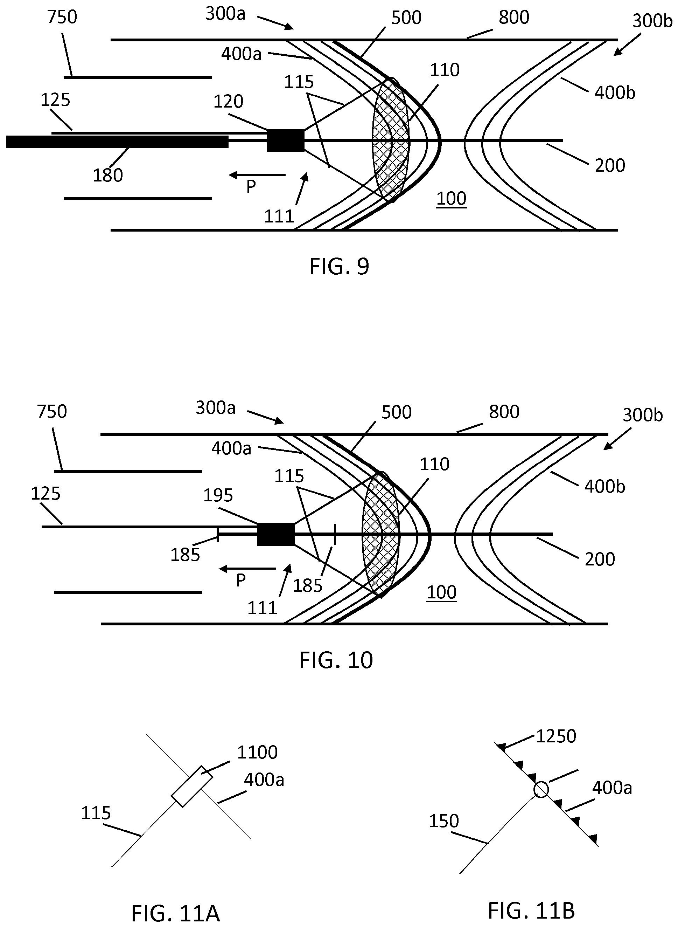

The linking elements 115 may be attached to the bristles by welding or adhesive, or by a heat shrinkable or meltable material, and the heat shrinkable material may be heated or melted to be secured to the bristles 400a. For example, as shown in Fig. 11, the linking elements 115 and one or more bristles 400a may extend through a heat-shrinkable material 1100. For example, the heat-shrinkable material may be a tube, with the linking element 115 extending through the lumen defined by the tube wall of the material 1100, and one or more bristles may extend through the tube wall. When heat is applied to the material 1100, the material 1100 shrinks to hold the linking element 115 and one or more bristles 400a securely. It is noted that any two components of the embolisation systems disclosed herein may be connected by such a heat shrinkable material, or via a meltable material which is melted around the components to securely attached them to each other.

The linking elements 115 may also be connected to a flow restricting membrane 500 attached to the core 200, having a collapsed delivery configuration and an expanded deployed configuration. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In the embodiment shown in Fig. 4A, the flow restricting membrane 500 is attached to one or more of the linking elements 115 (e.g. by welding or adhesive) and is therefore also retracted to a lower radial profile when the sliding element 120 is retracted. In other embodiments the flow restricting membrane 500 is not present or is not connected to the mesh 110, and may be configured to extend longitudinally in a distal direction.

Fig. 5A shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 5A, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

In the embodiment shown in Fig. 5A, the recapture mechanism 111 comprises a plurality of retractable elongate elements 150 which are each directly coupled to one or more of the bristles 400a at a point radially outwardly of the core 200. The elongate elements 150 may be slidably coupled or attached to the bristles 400a. For example, the elongate elements 150 may comprise loops slidably receiving one or more bristles 400a through the loops. For example, as shown in Fig. 11B, an elongate element 150 comprises a loop 1200 which slidably receives one or more bristles 400a. The elongate elements traverse the length of the delivery catheter 750 and are operable to be retracted by a user via a control mechanism.

The embolisation device 100 may comprise a detach element 140 configured to removably connect to a delivery element 135. The delivery element 135 is operable to deliver the embolisation device through the delivery catheter 750 as described with reference to Figs. 2A to 2C.

If the embolisation device 100 has been deployed in the bodily lumen 800 but must be recaptured, a user of the apparatus may actuate the recapture mechanism 111 by retracting the elongate elements 150 in the proximal direction P (and holding the delivery element 135 in place) which retracts the bristles 400a and causes the bristles 400a to be moved to a lower radial profile (shown in Fig. 5B). The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius of the delivery catheter 750 so that they do not abut the delivery catheter 750 when being recaptured. Again, once recaptured, the embolisation device 100 may be redeployed at a new position in the lumen 800. The recapture mechanism 111 may be reversed (i.e. advanced) to redeploy the bristles 400a or the bristles 400a may have a stiffness which reverse (i.e. advance) the recapture mechanism 111 such that the bristles are redeployed if the recapture mechanism 111 is not being actuated. The elongate elements 150 may be detached from the bristles 400a and the delivery element 135 may be detached from the detach element 140. The elongate elements 150/delivery element 135 may be removably connected to the bristles 400a/detach element 140 by any suitable mechanism. For example, as described above, they may be connected via a screw mechanism, or configured to break off from the bristles 400a/detach element 140 after a predetermined number of twists/rotations, or they may be attached to the bristles 400a/ detach element 140 via an electrolytic element configured to disintegrate upon application of an electric current through the elongate elements 150/delivery element 135. In embodiments where the elongate elements 150 are slidably connected to the bristles 400a, the elongate elements 150 may retract so that they slide off the bristles 400a the elongate elements 150 are removed from the embolisation device 100 when it is deployed.

The elongate elements 150 may each be attached to one or more of the bristles by welding or adhesive, or the elongate elements 150 may comprise a heat shrinkable or meltable material, and may be heated or melted to be secured to the bristles 400a.

The elongate elements 150 may also be connected to a flow restricting membrane 500 attached to the core 200, having a collapsed delivery configuration and an expanded deployed configuration. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In the embodiment shown in Fig. 5A, the flow restricting membrane 110 is attached to one or more of the elongate elements 150 (e.g. by welding or adhesive) and is therefore also retracted to a lower radial profile when the elongate elements are retracted. In other embodiments the flow restricting membrane 500 is not present or is not connected to the mesh 110, and may be configured to extend longitudinally in a distal direction.

In any of the embodiments of Figs. 2A to 3, the recapture mechanism 111 may be connected to the mesh 110 at a plurality of points distributed circumferentially about the mesh (for example the attachment points of the linking elements 115 or elongate elements 150 may be distributed about the mesh such that the retracting force of the mechanism is distributed about the mesh. This configuration avoids preferentially pulling the mesh 110 by the recapture mechanism 111 on one radial side of the embolisation device (for example if a single connection to the mesh exists on one radial side of the mesh).

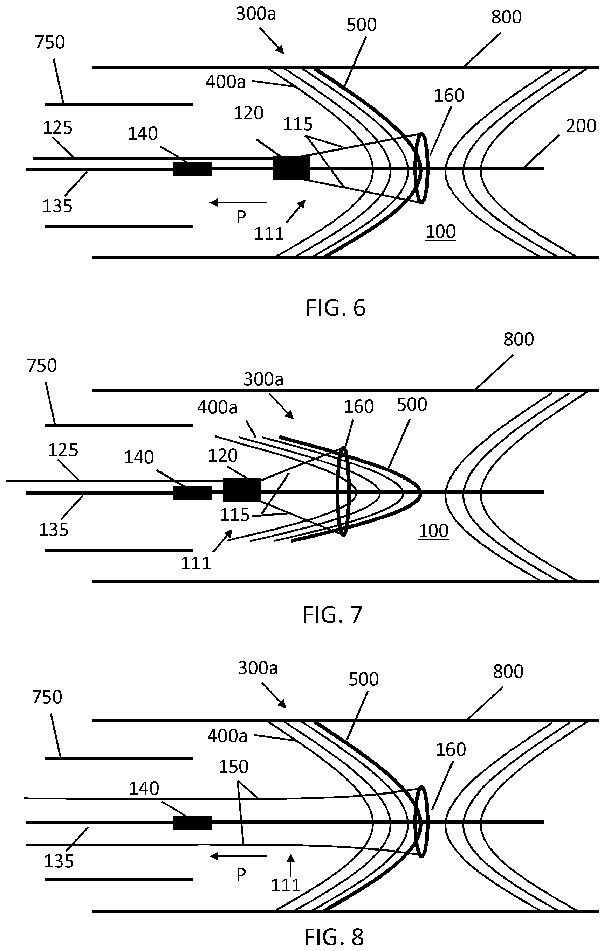

Fig. 6 shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 6, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

In the embodiment shown in Fig. 6, the apparatus comprises a collar 160 slidably mounted on the core 200 distally to the set of bristles 400a. The recapture mechanism 111 is configured to retract the collar 160 in the proximal direction. Doing so moves the collar 160 in a proximal direction, over the bristles 400a, which in turn causes the bristles to be push proximally and move to a lower radial profile. In some embodiments, the collar 160 may be a resiliently expandable material, for example an elastic material. The collar 160 may comprise materials such as Nylon and/or polyethylene.

In the embodiment shown in Fig. 6, the recapture mechanism 111 comprises a sliding element 120 slidably mounted on the core 200 proximal to the set of bristles 400a and coupled to the collar 160 via linking elements 115 (for example wires whose respective ends are attached to the sliding element 120 and the collar 160). The sliding element 120 may be, for example, a collar slidably mounted on the core 200. The sliding element 120 is connected to an elongate element 125 which extends through the delivery catheter 750 and is retractable by a user of the apparatus via a control mechanism.

Again, the embolisation device 100 may comprise a detach element 140 configured to removably connect to a delivery element 135. The delivery element 135 is operable to deliver the embolisation device through the delivery catheter 750 as described with reference to Figs. 2A to 2C.

If the embolisation device 100 has been deployed in the bodily lumen 800 (for example as shown in Fig. 6) and must be recaptured, a user of the apparatus may actuate the recapture mechanism 111 by retracting the elongate element 125 in the proximal direction P (and holding the delivery element 135 in place), which retracts the sliding element 120 and therefore the collar 160 via the linking elements 115. Doing so causes the bristles 400a to be moved to a lower radial profile as shown in Fig. 7. The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius of the delivery catheter 750 so that they do not abut the delivery catheter 750 when being recaptured.

Once recaptured, the embolisation device 100 may be redeployed at a new position in the lumen 800. The recapture mechanism 111 may be reversed (i.e. advanced) to redeploy the bristles 400a or the bristles 400a may have a stiffness which reverse (i.e. advance) the recapture mechanism 111 such that the bristles are redeployed if the recapture mechanism 111 is not being actuated. The elongate element 125 may be detached from the sliding element 120 and the delivery element 135 may be detached from the detach element 140. The elongate element 125/delivery element 135 may be removably connected to the sliding element 120/detach element 140 by any suitable mechanism. For example, as described above, they may be connected via a screw mechanism, or configured to break off from the sliding element 120/detach element 140 after a predetermined number of twists/rotations, or they may be attached to the sliding element 120/detach element 140 via an electrolytic element configured to disintegrate upon application of an electric current through the elongate element 125/delivery element 135.

Alternatively, the elongate element 125 may not be removable from the sliding element 120, and once the embolisation device 100 is correctly deployed, the elongate element can be completely retracted, the sliding element 120 and collar 160 sliding off the embolisation device (the sliding element 120 may be sized to slide over the detach element 140). Accordingly, the sliding element 120 and collar 160 may remain part of the implanted embolisation device 100 or may be removed completely from the implanted device.

In the embodiment shown in Fig. 6, the embolisation device 100 comprises a flow restricting membrane 500. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In other embodiments the flow restricting membrane 500 is not present or is located distally to the collar 160, and may be configured to extend longitudinally in a distal direction.

Fig. 8 shows an apparatus comprising an embolisation device 100 for promoting clot formation in a bodily lumen 800. The embolisation device 100 is shown in an expanded deployed configuration. The embolisation device 100 comprises a core 200, more specifically a stem, and a plurality of flexible bristles 400a, 400b, extending outwardly from the core 200, the bristles 400a, 400b having a collapsed delivery configuration and an expanded deployed configuration in which the bristles 400a, 400b extend at least radially outwardly from the core 200 to contact the lumen 800 and to anchor the device in the lumen 800. The outer portion of the bristles 400a, 400b contact the wall of the lumen 800. The embolisation device 100 is configured to be delivered in a distal direction from a delivery catheter 750. In the expanded deployed configuration shown in Fig. 8, a set of flexible bristles 400a extend radially outwardly from the core 200 and longitudinally in a proximal direction P opposite to the distal direction. The apparatus further comprises a recapture mechanism 111 for moving the set of flexible bristles 400a to a lower radial profile than in the expanded deployed configuration. More particularly, the recapture mechanism 111 moves the set of flexible bristles 400a to the lower radial profile before the delivery catheter 750 is moved over the set of flexible bristles 400a. As such, the recapture mechanism 111 is an active recapture mechanism actuatable independently of the movement of the delivery catheter 750 over the bristles 400a. It is noted that the embolisation device 100 may comprise any of the features of the examples described with reference to Figs. 1A to 1F (type of core, any number of bristle segments, flow restrictor, type of joint between bristle segments and so on). The distally-extending bristles 400b and the flow restrictor 500 may be considered as optional.

In the embodiment shown in Fig. 8, the apparatus comprises a collar 160 slidably mounted on the core 200 distally to the set of bristles 400a. The recapture mechanism 111 is configured to retract the collar 160 in the proximal direction. Doing so moves the collar 160 in a proximal direction, over the bristles 400a, which in turn causes the bristles to be pushed proximally and move to a lower radial profile. In some embodiments, the collar 160 may be a resiliently expandable material, for example an elastic material. The collar 160 may comprise materials such as Nylon and/or polyethylene.

In the embodiment shown in Fig. 8, the recapture mechanism 111 comprises one or more retractable elongate elements 150 attached to the collar 160. The elongate elements 150 traverse the length of the delivery catheter 750 and are retractable by a user via a control mechanism.

Again, the embolisation device 100 may comprise a detach element 140 configured to removably connect to a delivery element 135. The delivery element 135 is operable to deliver the embolisation device through the delivery catheter 750 as described with reference to Figs. 2A to 2C.

If the embolisation device 100 has been deployed in the bodily lumen 800 (for example as shown in Fig. 8) and must be recaptured, a user of the apparatus may actuate the recapture mechanism 111 by retracting the elongate elements 150 in the proximal direction P (and holding the delivery element 135 in place), which retracts the collar 160. Doing so causes the bristles 400a to be moved to a lower radial profile, similar to that shown in Fig. 7. The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius of the delivery catheter 750 so that they do not abut the delivery catheter 750 when being recaptured.

Once recaptured, the embolisation device 100 may be redeployed at a new position in the lumen 800. The recapture mechanism 111 may be reversed (i.e. advanced) to redeploy the bristles 400a or the bristles 400a may have a stiffness which reverse (i.e. advance) the recapture mechanism 111 such that the bristles are redeployed when the recapture mechanism 111 is not being actuated. The elongate elements 150 may be detached from the collar 160 and the delivery element 135 may be detached from the detach element 140. The elongate elements 150/delivery element 135 may be removably connected to the collar 160/detach element 140 by any suitable mechanism. For example, as described above, they may be connected via a screw mechanism, or configured to break off from the collar 160/detach element 140 after a predetermined number of twists/rotations, or they may be attached to the collar 160/detach element 140 via an electrolytic element configured to disintegrate upon application of an electric current through the elongate elements 150/delivery element 135.

Alternatively, the elongate elements 150 may not be removable from the collar 160, and once the embolisation device 100 is correctly deployed, the elongate elements 150 can be completely retracted, the collar 160 sliding off the embolisation device 100. Accordingly, the collar 160 may remain part of the implanted embolisation device 100 or may be removed completely from the implanted device.

In the embodiment shown in Fig. 8, the embolisation device 100 comprises a flow restricting membrane 500. In the expanded deployed configuration, the flow restricting membrane 500 extends radially outwardly and longitudinally in a proximal direction P opposite to the distal direction, and the recapture mechanism 111 may also be configured to move the flow restricting membrane 500 to a lower radial profile than in the expanded deployed configuration. In other embodiments the flow restricting membrane 500 is not present or is located distally to the collar 160, and may be configured to extend longitudinally in a distal direction.

In the embodiments shown Figs. 6 to 8, the recapture mechanism 111 may be connected to the collar 160 as a plurality of points distributed circumferentially about the collar 160 (for example the attachment points of the linking elements 115 or elongate elements 150 may be distributed about the core such that the retracting force of the mechanism is distributed about the collar 160). This configuration avoids preferentially pulling the collar by the recapture mechanism 111 on one radial side of the embolisation device (for example if a single connection to the collar exists on one radial side of the collar).

In any of the embodiments disclosed herein, the some or all of the bristles may comprise hooks or other protrusions 1250 (see Fig. 11B) which increase the surface friction of the bristles, inhibiting the retracting mechanism from becoming disconnected from the bristles.

Whilst in the illustrated embodiments, the embolisation device 100 is shown as having a detach element 140 at a proximal end of the embolisation device 100, it will be appreciated that in other embodiments the embolisation device may not comprise a detach element. For example, in any of the embodiments described herein, the embolisation device may not comprise detach element 140 and the embolisation device 100 may be pushed through the delivery catheter 750 using a pusher 180, which abuts the proximal end of the embolisation device 100, but which is not attached to the embolisation device (for example as shown in Fig. 9).

Furthermore, in any of the embodiments described herein, the apparatus may comprise a sliding detach element 195 which is both the detach element 140 and sliding element 120, as illustrated in Fig. 10. In particular, the sliding element 140 of any embodiment, in conjunction with the elongate element 125, may be used to deliver the embolisation device 100 to the body lumen 800. The embolisation device may comprise stopping elements 185 (e.g. protrusions or sections of core 200 of a larger diameter) distally and/or proximally to the sliding detach element 195 to restrict the extent which the sliding detach element 195 may translate along the core 200.

The elongate element 125 is operable to push the embolisation device 100 through the delivery catheter 750. The embolisation device 100 is deployed to the expanded deployed configuration by moving the elongate element 125 distally relative to the delivery catheter 750 (for example by holding the delivery catheter 750 and pushing the elongate element 125 or by holding the elongate element 125 and pulling the delivery catheter 750). If the embolisation device 100 has been deployed in the bodily lumen 800 (for example as shown in Fig. 10) and must be recaptured, a user of the apparatus may actuate the recapture mechanism 111 by retracting the elongate element 125 in the proximal direction P, which retracts the sliding element 120 and therefore the mesh 110 via the linking elements 115. In particular, the retracting mechanism is still able to be retracted due to the opposing anchoring force of the deployed embolisation device within the lumen 800. Doing so causes the bristles 400a to be moved to a lower radial profile. The delivery catheter 750 may then be advanced over the bristles 400a without causing damage to the bristles 400a or reorienting them. The recapture mechanism 111 may be configured to decrease the radial profile of the set of flexible bristles 400a to a radial profile smaller than an inner radius R of the delivery catheter 750 so that they do not abut the delivery catheter 750 when being recaptured. Once recaptured, the embolisation device 100 may be redeployed at a new position in the lumen 800. The recapture mechanism 111 may be reversed (i.e. advanced) to redeploy the bristles 400a or the bristles 400a may have a stiffness which reverse (i.e. advance) the recapture mechanism 111 such that the bristles are redeployed if the recapture mechanism 111 is not being actuated. The elongate element 1125 may be detached as described in the above embodiments. Further, if proximal protrusion 185 is not provided, all of the components of the recapture mechanism 111 (e.g. including the mesh 110 or the collar 160) may be completely removed from the implanted device, as described in the embodiments above.

The apparatuses disclosed herein may be provided by the following method:
- providing a core;
- providing a set of flexible bristles configured to extend radially outwardly from the core and longitudinally in a proximal direction opposite to a direction in which the embolisation device is configured to be delivered from a delivery catheter; and
- providing a recapture mechanism for moving the set of bristles to a lower radial profile than an expanded deployed configuration of the set of bristles.

The step of providing a recapture mechanism may comprise slidably or fixably connecting the set of bristles to the recapture mechanism

The recapture mechanism may be connected to the bristles by a heat shrinkable material (for example the elongate elements 150 or linking elements 115 may be connected to the bristles 400 and/or mesh 110 by a heat shrinkable material analogous to the configuration shown in Fig. 11A) and the step of providing the recapture mechanism may comprise slidably receiving the set of bristles through the heat shrinkable material, and heat shrinking the material. Alternatively, the recapture mechanism may be connected to the bristles by a meltable material and the step of providing the recapture mechanism may comprise slidably receiving the set of bristles through the meltable material, and melting the material.

As has been described above, it will be appreciated that there are numerous formulations of the recapture mechanism. It will be appreciated that the recapture mechanism can be any mechanism which is configured to move the set of proximally-extending bristles to a lower radial profile than in the expanded deployed configuration before the delivery catheter is moved over the set of bristles.

All of the above are fully within the scope of the present disclosure and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. An apparatus comprising:
an embolisation device (100) for promoting clot formation in a bodily lumen (800), the embolisation device (100) comprising a core (200) and a plurality of flexible bristles (400a, 400b) extending outwardly from the core (200), the bristles (400a, 400b) having a collapsed delivery configuration and an expanded deployed configuration in which the bristles (400a, 400b) extend at least radially outwardly from the core (200) to contact the lumen (800) and to anchor the device (100) in the lumen (800), and
a delivery catheter (750), wherein:
the embolisation device (100) is configured to be delivered in a distal direction from the delivery catheter (750) to the lumen (800);
in the expanded deployed configuration, a set of the flexible bristles (400a) extend radially outwardly from the core (200) and longitudinally in a proximal direction opposite to the distal direction; and
the apparatus further comprises a recapture mechanism (111),
**characterised in that**
the recapture mechanism (111) is actuatable independently of the movement of the delivery catheter (750) over the bristles (400a, 400b), for moving the set of flexible bristles (400a) to a lower radial profile than in the expanded deployed configuration before the delivery catheter (750) is moved over the set of flexible bristles (400a).

2. The apparatus of claim 1, wherein the embolisation device (100) comprises a detach element (140, 195) at a proximal end of the embolisation device (100), the detach element (140, 195) configured to removably connect to a delivery element (135) for delivering the embolisation device (100) to a bodily lumen (800) through the delivery catheter (750), optionally wherein the recapture mechanism (111) comprises a sliding element (120) mounted on the core (200) proximal to the set of bristles (400a) and coupled to the set of flexible bristles (400a), such that moving the sliding element (120) in the proximal direction moves the set of flexible bristles (400a) to a lower radial profile.

3. The apparatus of claim 1, wherein the recapture mechanism (111) comprises a sliding element (120) mounted on the core (200) proximal to the set of bristles (400a) and coupled to the set of flexible bristles (400a), such that moving the sliding element (120) in the proximal direction moves the set of flexible bristles (400a) to a lower radial profile.

4. The apparatus of claim 3, wherein the sliding element (120) is a detach element (195) configured to removably connect to a delivery element (135) for delivering the embolisation device (100) to a bodily lumen (800) through the delivery catheter (750).

5. The apparatus of any preceding claim, wherein the recapture mechanism (111) comprises one or more retractable elongate elements (150) each coupled to one or more bristles of the set (400a), such that retracting the elongate elements (150) in the proximal direction moves the set of flexible bristles (400a) to the lower radial profile.

6. The apparatus of claim 5, wherein the retractable elongate elements (150) are slidably mounted or attached to the bristles in the set (400a).

7. The apparatus of any preceding claim, wherein the embolisation device (100) comprises a mesh (110) mounted on the core (200) and connected to the set of bristles (400a) radially outwardly from the core (200); and the recapture mechanism (111) is configured to retract the mesh (110) in the proximal direction to move the set of bristles (400a) to the lower radial profile.

8. The apparatus of claim 7, wherein the mesh (110) comprises a meltable or heat-shrinkable material which surrounds and secures the bristles in the set (400a) radially outwardly from the core (200).

9. The apparatus of claim 7, wherein the set of bristles (400a) are slidably received by the mesh (110) radially outwardly from the core (200).

10. The apparatus of any of claims 1 to 6, wherein the embolisation device (100) comprises a collar (160) slidably mounted on the core (200) distal to the set of bristles (400a), and the recapture mechanism (111) configured to retract the collar (160) in the proximal direction, such that retracting the collar (160) by the recapture mechanism (111) moves the set of bristles (400a) to the lower radial profile.

11. The apparatus of claim 10, wherein the recapture mechanism (111) is connected to the collar (160) at a plurality of points distributed circumferentially about the collar (160); or
the apparatus of any of claims 7 to 9, wherein the recapture mechanism (111) is connected to the mesh (110) at a plurality of points distributed circumferentially about the mesh (110).

12. The apparatus of any preceding claim, wherein a plurality of flexible bristles in the set (400a) comprise protrusions (1250) on the bristles for increasing the surface friction of the bristles.

13. The apparatus of any preceding claim, further comprising a flow restricting membrane (50) on the core (200) having a collapsed delivery configuration and an expanded deployed configuration; optionally wherein in the expanded deployed configuration, the flow restricting membrane (50) extends radially outwardly and longitudinally in a proximal direction opposite to the distal direction, and the recapture mechanism (111) is configured to move the flow restricting membrane (50) to a lower radial profile than in the expanded deployed configuration.

14. An embolisation system comprising:
the apparatus of any preceding claim; and
a delivery element (135) for delivering the embolisation device (100) to a bodily lumen (800) through the delivery catheter (750);
wherein the recapture mechanism (111) is actuatable to decrease the radial profile the set of flexible bristles (400a) to a radial profile smaller than an inner radius of the distal tip of the delivery catheter (750).

15. A method of manufacturing an apparatus comprising an embolisation device (100) for promoting clot formation in a bodily lumen (800), the embolisation device (100) comprising a core (200) and a plurality of flexible bristles (400a, 400b) extending outwardly from the core (200), the bristles (400a, 400b) having a collapsed delivery configuration and an expanded deployed configuration in which the bristles (400a, 400b) extend at least radially outwardly from the core (200) to contact the lumen (800) and to anchor the device (100) in the lumen (800), the method comprising:
providing a delivery catheter (750);
providing a core (200);
providing a set of flexible bristles (400a) configured to extend radially outwardly from the core (200) and longitudinally in a proximal direction opposite to a direction in which the embolisation device (100) is configured to be delivered from a delivery catheter (750); and
providing a recapture mechanism (111),
**characterised in that**
the recapture mechanism (111) is actuatable independently of the movement of the delivery catheter (750) over the bristles (400a, 400b), for moving the set of bristles (400a) to a lower radial profile than an expanded deployed configuration of the set of bristles (400a) before the delivery catheter (750) is moved over the set of flexible bristles (400a).

## Patentansprüche

1. Einrichtung, umfassend:
eine Embolisierungsvorrichtung (100) zum Fördern der Gerinnselbildung in einem Körperlumen (800), die Embolisierungsvorrichtung (100) umfassend einen Kern (200) und eine Vielzahl von flexiblen Borsten (400a, 400b), die sich vom Kern (200) nach außen erstrecken, wobei die Borsten (400a, 400b) eine zusammengefaltete Zuführkonfiguration und eine expandierte, eingesetzte Konfiguration aufweisen, in der sich die Borsten (400a, 400b) vom Kern (200) mindestens radial nach außen erstrecken, um das Lumen (800) zu berühren und die Vorrichtung (100) im Lumen (800) zu verankern, und
einen Zuführkatheter (750), wobei:
die Embolisierungsvorrichtung (100) dazu konfiguriert ist, in einer distalen Richtung vom Zuführkatheter (750) zum Lumen (800) zugeführt zu werden;
in der expandierten, eingesetzten Konfiguration, ein Satz von den flexiblen Borsten (400a) sich vom Kern (200) radial nach außen und in Längsrichtung in einer proximalen Richtung entgegengesetzt zur distalen Richtung erstreckt; und
die Einrichtung weiter einen Rückholmechanismus (111) umfasst,
**dadurch gekennzeichnet, dass**
der Rückholmechanismus (111) unabhängig von der Bewegung des Zuführkatheters (750) über die Borsten (400a, 400b) betätigbar ist, um den Satz von flexiblen Borsten (400a) zu einem niedrigeren radialen Profil als in der expandierten, eingesetzten Konfiguration zu bewegen, bevor der Zuführkatheter (750) über den Satz von flexiblen Borsten (400a) bewegt wird.

2. Einrichtung nach Anspruch 1, wobei die Embolisierungsvorrichtung (100) ein Ablöseelement (140, 195) an einem proximalen Ende der Embolisierungsvorrichtung (100) umfasst, wobei das Ablöseelement (140, 195) dazu konfiguriert ist, entfernbar mit einem Zuführelement (135) zu verbinden, um die Embolisierungsvorrichtung (100) durch den Zuführkatheter (750) einem Körperlumen (800) zuzuführen, optional, wobei der Rückholmechanismus (111) ein Gleitelement (120) umfasst, das auf dem Kern (200) proximal zum Satz von Borsten (400a) montiert ist und mit dem Satz von flexiblen Borsten (400a) gekoppelt ist, sodass eine Bewegung des Gleitelements (120) in proximaler Richtung den Satz von flexiblen Borsten (400a) zu einem niedrigeren radialen Profil bewegt.

3. Einrichtung nach Anspruch 1, wobei der Rückholmechanismus (111) ein Gleitelement (120) umfasst, das auf dem Kern (200) proximal zum Satz von Borsten (400a) montiert ist und mit dem Satz von flexiblen Borsten (400a) gekoppelt ist, sodass eine Bewegung des Gleitelements (120) in proximaler Richtung den Satz von flexiblen Borsten (400a) zu einem niedrigeren radialen Profil bewegt.

4. Einrichtung nach Anspruch 3, wobei das Gleitelement (120) ein Ablöseelement (195) ist, das dazu konfiguriert ist, entfernbar mit einem Zuführelement (135) zu verbinden, um die Embolisierungsvorrichtung (100) durch den Zuführkatheter (750) einem Körperlumen (800) zuzuführen.

5. Einrichtung nach einem vorstehenden Anspruch, wobei der Rückholmechanismus (111) ein oder mehrere zurückziehbare längliche Elemente (150) umfasst, die jeweils mit einer oder mehreren Borsten des Satzes (400a) gekoppelt sind, sodass ein Zurückziehen der länglichen Elemente (150) in proximaler Richtung den Satz von flexiblen Borsten (400a) zu dem niedrigeren radialen Profil bewegt.

6. Einrichtung nach Anspruch 5, wobei die zurückziehbaren länglichen Elemente (150) verschiebbar an den Borsten in dem Satz (400a) montiert oder angebracht sind.

7. Einrichtung nach einem vorstehenden Anspruch, wobei die Embolisierungsvorrichtung (100) ein Netz (110) umfasst, das auf dem Kern (200) montiert ist und mit dem Satz von Borsten (400a) vom Kern (200) radial nach außen verbunden ist; und der Rückholmechanismus (111) dazu konfiguriert ist, das Netz (110) in proximaler Richtung zurückzuziehen, um den Satz von Borsten (400a) zu dem unteren radialen Profil zu bewegen.

8. Einrichtung nach Anspruch 7, wobei das Netz (110) ein schmelzbares oder wärmeschrumpfbares Material umfasst, das die Borsten im Satz (400a) vom Kern (200) radial nach außen umgibt und fixiert.

9. Einrichtung nach Anspruch 7, wobei der Satz von Borsten (400a) durch das Netz (110) vom Kern (200) radial nach außen verschiebbar aufgenommen ist.

10. Einrichtung nach einem der Ansprüche 1 bis 6, wobei die Embolisierungsvorrichtung (100) einen Kragen (160) umfasst, der verschiebbar auf dem Kern (200) distal zum Satz von Borsten (400a) montiert ist, und der Rückholmechanismus (111) dazu konfiguriert ist, den Kragen (160) in proximaler Richtung zurückzuziehen, sodass das Zurückziehen des Kragens (160) durch den Rückholmechanismus (111) den Satz von Borsten (400a) zu dem niedrigeren radialen Profil bewegt.

11. Einrichtung nach Anspruch 10, wobei der Rückholmechanismus (111) mit dem Kragen (160) an einer Vielzahl von Punkten verbunden ist, die in Umfangsrichtung um den Kragen (160) verteilt sind; oder
die Einrichtung nach einem der Ansprüche 7 bis 9, wobei der Rückholmechanismus (111) mit dem Netz (110) an einer Vielzahl von Punkten verbunden ist, die in Umfangsrichtung um das Netz (110) verteilt sind.

12. Einrichtung nach einem vorstehenden Anspruch, wobei eine Vielzahl von flexiblen Borsten im Satz (400a) Vorsprünge (1250) an den Borsten umfasst, um die Oberflächenreibung der Borsten zu erhöhen.

13. Einrichtung nach einem vorstehenden Anspruch, weiter umfassend eine durchflussbegrenzende Membran (50) auf dem Kern (200), die eine zusammengefaltete Zuführkonfiguration und eine expandierte, eingesetzte Konfiguration aufweist; optional, wobei sich die durchflussbegrenzende Membran (50) in der expandierten, eingesetzten Konfiguration radial nach außen und in Längsrichtung in einer proximalen Richtung entgegengesetzt zur distalen Richtung erstreckt, und der Rückholmechanismus (111) dazu konfiguriert ist, die durchflussbegrenzende Membran (50) zu einem niedrigeren radialen Profil als in der expandierten, eingesetzten Konfiguration zu bewegen.

14. Embolisierungssystem, umfassend:
die Einrichtung nach einem vorstehenden Anspruch; und
ein Zuführelement (135) zum Zuführen der Embolisierungsvorrichtung (100) durch den Zuführkatheter (750) zu einem Körperlumen (800);
wobei der Rückholmechanismus (111) betätigbar ist, um das radiale Profil des Satzes von flexiblen Borsten (400a) auf ein radiales Profil zu verringern, das kleiner ist als ein Innenradius der distalen Spitze des Zuführkatheters (750).

15. Verfahren zum Herstellen einer Einrichtung, umfassend eine Embolisierungsvorrichtung (100) zum Fördern der Gerinnselbildung in einem Körperlumen (800), die Embolisierungsvorrichtung (100) umfassend einen Kern (200) und eine Vielzahl von flexiblen Borsten (400a, 400b), die sich vom Kern (200) nach außen erstrecken, wobei die Borsten (400a, 400b) eine zusammengefaltete Zuführkonfiguration und eine expandierte, eingesetzte Konfiguration aufweisen, in der sich die Borsten (400a, 400b) vom Kern (200) mindestens radial nach außen erstrecken, um das Lumen (800) zu berühren und die Vorrichtung (100) im Lumen (800) zu verankern, das Verfahren umfassend:
Bereitstellen eines Zuführkatheters (750);
Bereitstellen eines Kerns (200);
Bereitstellen eines Satzes von flexiblen Borsten (400a), die dazu konfiguriert sind, sich vom Kern (200) radial nach außen und in Längsrichtung in einer proximalen Richtung entgegengesetzt zu einer Richtung zu erstrecken, in der die Embolisierungsvorrichtung (100) dazu konfiguriert ist, von einem Zuführkatheter (750) zugeführt zu werden; und
Bereitstellen eines Rückholmechanismus (111),
**dadurch gekennzeichnet, dass**
der Rückholmechanismus (111) unabhängig von der Bewegung des Zuführkatheters (750) über die Borsten (400a, 400b) betätigbar ist, um den Satz von Borsten (400a) zu einem niedrigeren radialen Profil als einer expandierten, eingesetzten Konfiguration des Satzes von Borsten (400a) zu bewegen, bevor der Zuführkatheter (750) über den Satz von flexiblen Borsten (400a) bewegt wird.

## Revendications

1. Appareil comprenant :
un dispositif (100) d'embolisation pour favoriser la formation de caillots dans une lumière corporelle (800), le dispositif (100) d'embolisation comprenant une partie centrale (200) et une pluralité de poils souples (400a, 400b) s'étendant vers l'extérieur à partir de la partie centrale (200),
les poils (400a, 400b) présentant une configuration de pose repliée et une configuration déployée étendue dans laquelle les poils (400a, 400b) s'étendent au moins radialement vers l'extérieur à partir de la partie centrale (200) pour entrer en contact avec la lumière (800) et pour ancrer le dispositif (100) dans la lumière (800), et
un cathéter (750) de pose, dans lequel :
le dispositif (100) d'embolisation est configuré pour être acheminé dans une direction distale du cathéter (750) de pose vers la lumière (800) ;
dans la configuration déployée étendue, un ensemble des poils souples (400a) s'étend radialement vers l'extérieur à partir de la partie centrale (200) et longitudinalement dans une direction proximale opposée à la direction distale ; et
l'appareil comprend en outre un mécanisme (111) de récupération, **caractérisé en ce que**
le mécanisme (111) de récupération est actionnable indépendamment du déplacement du cathéter (750) de pose sur les poils (400a, 400b), pour déplacer l'ensemble de poils souples (400a) vers un profil radial inférieur à celui dans la configuration déployée étendue avant que le cathéter (750) de pose ne soit déplacé sur l'ensemble de poils souples (400a).

2. Appareil selon la revendication 1, dans lequel le dispositif (100) d'embolisation comprend un élément détachable (140, 195) sur une extrémité proximale du dispositif (100) d'embolisation, l'élément détachable (140, 195) étant configuré pour se lier de manière amovible à un élément (135) de pose pour acheminer le dispositif (100) d'embolisation vers une lumière corporelle (800) à travers le cathéter (750) de pose, éventuellement dans lequel le mécanisme (111) de récupération comprend un élément coulissant (120) monté sur la partie centrale (200) à proximité de l'ensemble de poils (400a) et couplé à l'ensemble de poils souples (400a), de sorte que le déplacement de l'élément coulissant (120) dans la direction proximale déplace l'ensemble de poils souples (400a) vers un profil radial inférieur.

3. Appareil selon la revendication 1, dans lequel le mécanisme (111) de récupération comprend un élément coulissant (120) monté sur la partie centrale (200) à proximité de l'ensemble de poils (400a) et couplé à l'ensemble de poils souples (400a), de sorte que le déplacement de l'élément coulissant (120) dans la direction proximale déplace l'ensemble de poils souples (400a) vers un profil radial inférieur.

4. Appareil selon la revendication 3, dans lequel l'élément coulissant (120) est un élément détachable (195) configuré pour se lier de manière amovible à un élément (135) de pose destiné à acheminer le dispositif (100) d'embolisation vers une lumière corporelle (800) à travers le cathéter (750) de pose.

5. Appareil selon une quelconque revendication précédente, dans lequel le mécanisme (111) de récupération comprend un ou plusieurs éléments allongés rétractables (150), chacun couplé à un ou plusieurs poils de l'ensemble (400a), de sorte que la rétraction des éléments allongés (150) dans la direction proximale déplace l'ensemble de poils souples (400a) vers le profil radial inférieur.

6. Appareil selon la revendication 5, dans lequel les éléments allongés rétractables (150) sont montés sur ou fixés aux poils dans l'ensemble (400a) de manière coulissante.

7. Appareil selon une quelconque revendication précédente, dans lequel le dispositif (100) d'embolisation comprend un maillage (110) monté sur la partie centrale (200) et relié à l'ensemble de poils (400a) radialement vers l'extérieur à partir de la partie centrale (200) ; et le mécanisme (111) de récupération est configuré pour rétracter le maillage (110) dans la direction proximale pour déplacer l'ensemble de poils (400a) vers le profil radial inférieur.

8. Appareil selon la revendication 7, dans lequel le maillage (110) comprend un matériau fusible ou thermorétractable qui entoure et fixe les poils dans l'ensemble (400a) radialement vers l'extérieur à partir de la partie centrale (200).

9. Appareil selon la revendication 7, dans lequel l'ensemble de poils (400a) est reçu de manière coulissante par le maillage (110) radialement vers l'extérieur à partir de la partie centrale (200).

10. Appareil selon l'une quelconque des revendications 1 à 6, dans lequel le dispositif (100) d'embolisation comprend un collier (160) monté de manière coulissante sur la partie centrale (200) distale par rapport à l'ensemble de poils (400a), et le mécanisme (111) de récupération étant configuré pour rétracter le collier (160) dans la direction proximale, de telle sorte que la rétraction du collier (160) par le mécanisme (111) de récupération déplace l'ensemble de poils (400a) vers le profil radial inférieur.

11. Appareil selon la revendication 10, dans lequel le mécanisme (111) de récupération est relié au collier (160) en une pluralité de points répartis de manière circonférentielle autour du collier (160) ; ou appareil selon l'une quelconque des revendications 7 à 9, dans lequel le mécanisme (111) de récupération est relié au maillage (110) en une pluralité de points répartis de manière circonférentielle autour du maillage (110).

12. Appareil selon une quelconque revendication précédente, dans lequel une pluralité de poils souples dans l'ensemble (400a) comprennent des saillies (1250) sur les poils pour augmenter le frottement de surface des poils.

13. Appareil selon une quelconque revendication précédente, comprenant en outre une membrane (50) de limitation d'écoulement sur la partie centrale (200) présentant une configuration de pose repliée et une configuration déployée étendue ; éventuellement, dans lequel dans la configuration déployée étendue, la membrane (50) de limitation d'écoulement s'étend radialement vers l'extérieur et longitudinalement dans une direction proximale opposée à la direction distale, et le mécanisme (111) de récupération est configuré pour déplacer la membrane (50) de limitation d'écoulement vers un profil radial inférieur à celui dans la configuration déployée étendue.

14. Système d'embolisation comprenant :
l'appareil selon une quelconque revendication précédente ; et
un élément (135) de pose pour acheminer le dispositif (100) d'embolisation vers une lumière corporelle (800) à travers le cathéter (750) de pose ;
dans lequel le mécanisme (111) de récupération est actionnable pour réduire le profil radial de l'ensemble de poils souples (400a) à un profil radial plus petit qu'un rayon interne de la pointe distale du cathéter (750) de pose.

15. Procédé de fabrication d'un appareil comprenant un dispositif (100) d'embolisation pour favoriser la formation de caillots dans une lumière corporelle (800), le dispositif (100) d'embolisation comprenant une partie centrale (200) et une pluralité de poils souples (400a, 400b) s'étendant vers l'extérieur à partir de la partie centrale (200), les poils (400a, 400b) présentant une configuration de pose repliée et une configuration déployée étendue dans laquelle les poils (400a, 400b) s'étendent au moins radialement vers l'extérieur à partir de la partie centrale (200) pour entrer en contact avec la lumière (800) et pour ancrer le dispositif (100) dans la lumière (800), le procédé comprenant :
la fourniture d'un cathéter (750) de pose ;
la fourniture d'une partie centrale (200) ;
la fourniture d'un ensemble de poils souples (400a) configuré pour s'étendre radialement vers l'extérieur à partir de la partie centrale (200) et longitudinalement dans une direction proximale opposée à une direction dans laquelle le dispositif (100) d'embolisation est configuré pour être acheminé à partir d'un cathéter (750) de pose ; et
la fourniture d'un mécanisme (111) de récupération,
**caractérisé en ce que**
le mécanisme (111) de récupération est actionnable indépendamment du déplacement du cathéter (750) de pose sur les poils (400a, 400b), pour déplacer l'ensemble de poils (400a) vers un profil radial inférieur à celui d'une configuration déployée étendue de l'ensemble de poils (400a) avant que le cathéter (750) de pose ne soit déplacé sur l'ensemble de poils souples (400a).
